# EUROPEAN PATENT APPLICATION

(11) **EP 4 725 475 A1**
(43) Date of publication of application: **15.04.2026**
(21) Application number: 25206470.4
(22) Date of filing: 02.10.2025
(51) Int. Cl.: A61K 9/00, A61K 9/16, A61K 39/395, C07K 16/28, C07K 16/32

(54) **MICRONISED ANTIBODIES AND METHOD FOR THEIR OBTAINMENT**

(30) Priority: 14.10.2024 IT 202400022827
(71) Applicant: Munit SA, 6828 Balerna (CH); Fondazione IRCCS Istituto Nazionale dei Tumori, 20133 Milan (IT)
(72) Inventor: DAL BOSCO, Massimo, 6828 Balerna (CH); FIGINI, Mariangela, 20133 MILANO (IT); FRIGERIO, Giovanni, 6828 Balerna (CH); LUISON, Elena, 20133 MILANO (IT)
(74) Representative: Merli, Silvia

(57) **Abstract**

It is the object of the present invention a method of making a powder for inhalation comprising the step of:
- Granulating an un-milled carrier with a solution comprising at least one monoclonal antibody (mAb) to obtain a dried granulated mixture;
- Micronizing the dried granulated mixture with a spiral jet mill, obtaining
- a micronized powder.

## Description

### Background

Dry powder inhaler (DPI) formulation is a valid alternative to the metered dose inhalers (MDI) formulation to administrate mAb (monoclonal antibodies) into the lungs or other site reached by inhalation. Direct pulmonary delivery of antibodies is an important aspect to achieve local inhibition of targets of interest. To achieve these results, a particle size in the low micrometres range is required.

Jet milling is a process to micronize particles. The milling energy is the kinetic energy supplied by gaseous fluid jets (filtered compressed nitrogen) having known speed. A particle enters the milling chamber at a speed V1, gets a circular movement and it is accelerated, by the injector nozzles, to a higher speed V2. When two rotating particles collide at speed V1 and V2, their kinetic energy breaks them into numerous smaller ones. The number of particles building up after collision and their size, namely, their mass, depend directly on the kinetic energy accumulated by the two particles before the collision. In conclusion, the particle size can be regulated by controlling jets speed and distance between particles before the collision. Therefore, the parameters controlling the final particle size distribution are:
- amount of product fed into the milling chamber per unit time (feed rate): varying the feed rate, particles concentration inside the milling chamber changes, hence distance covered by two particles before the collision is influenced.
- pressure: varying the fluid pressure through the nozzle section, that remains constant, the jet speed changes.

Micronization of monoclonal antibody was demonstrated to be possible. As an example, EP0562125 describes a composition containing an antibody obtained by freeze-drying a solution of an antibody followed by milling, having a particle diameter of more than 0,5 µm and less than 10 µm, i.e., a lyophilized powder is micronized.

The challenge is to obtain a micronized powder with the flow property required to allow the mixing of said micronized mAb with the desired excipients to generate a dry powder suitable for DPI formulation, avoiding the expensive lyophilization step.

### Description

It forms an object of the present invention a method to generate a homogenously blended mAb into an excipient, with the suitable particle size and flow property to be used for DPI device, starting from a mAb solution.

In an embodiment, said mAb is solubilised in its pharmaceutical formulation.

The method according to the present invention is performed at lower process cost compared to the state of the art, and it is capable to generate a blended micronized powder suitable for DPI application.

### Drawings description

**Figure 1****:** schematic representation of a micronization facility.
**Figure 2****:** SDS-PAGE on acrylamide gel 4-12%. Samples: 1= Liquid Trastuzumab on lactose; 2= sample 1; 3= sample 2; M= Marker. To note, sample 1 and sample 2 are the same samples, resuspended in two separated vials.
**Figure 3****:** SEC Chromatogram performed with A) molecular weight standard MWS B) Lactose C) Liquid Trastuzumab in lactose D) Sample 1 and E) Sample 2.
**Figure 4****:** Biacore sensogram of: Liquid Trastuzumab 25 nM, Sample 1, 25 nM and Sample 2, 25 nM on rHER2-ECD.
**Figure 5****:** FACS analysis. Reactivity of liquid Trastuzumab in lactose and samples 1 and 2 on A) positive (BT474) and B) negative (MDAMB468) cell line.
**Figure 6****:** Binding, evaluated in ELISA at different concentrations (from 10 to 0.01 µg/ml), on positive (MDAMB361) and negative (MDAMB468) cells lines.
**Figure 7****:** SDS-PAGE on acrylamide gel 4-12% Samples: 1= Trastuzumab from powder; 2= Sample 1; 3= Sample 2; M= Marker.
**Figure 8****:** SEC Chromatogram performed with A) MWS B) Trastuzumab from powder C) Sample 1 and D) Sample 2.
**Figure 9****:** FACS analysis. A) Reactivity of Trastuzumab from powder and Samples 1 and 2 on A) Positive (MDAMB361) and B) negative (MDAMB468) cell lines.
**Figure 10****:** Binding, evaluated in ELISA at different concentrations (from 10 to 0.0025 µg/ml), on positive (MDAMB361) and negative (MDAMB468) cell lines.
**Figure 11****:** Biacore Sensorgrams of: Trastuzumab in lactose 20 nM, Sample 1 20 nM and Sample 2 20 nM on rHER2-ECD.
**Figure 12****:** SDS-PAGE on acrylamide gel 4-12% Samples: 1= Rituximab loaded at 10 mg\ml on lactose; 2= Sample 1; 3= Sample 2; M= Marker.
**Figure 13****:** SEC Chromatogram performed with A) MWS B) Rituximab loaded at 10 mg\ml on lactose C) Sample 1 D) Sample 2 and E) Lactose.
**Figure 14****:** FACS analysis. A) Reactivity of Rituximab loaded at 10 mg\ml and Samples on A) Positive (RAJI) and B) negative (JURKAT) cell lines.
**Figure 15****:** SDS-PAGE on acrylamide gel 4-12% Samples: 1= Rituximab loaded at 20 mg\ml on lactose; 2= Sample 1; 3= Sample 2; M= Marker.
**Figure 16****:** SEC Chromatogram performed with A) MWS B) Rituximab loaded at 20 mg\ml on lactose C) Sample 1 D) Sample 2 and E) Lactose.
**Figure 17****:** FACS analysis. A) Reactivity of Rituximab loaded at 20 mg\ml and Samples on A) Positive (RAJI) and B) negative (JURKAT) cell line.

### Detailed description

The method according to the present invention comprises:
- Granulating an un-milled carrier with a solution comprising at least one mAb to obtain a dried granulated mixture;
- Micronizing the dried granulated mixture with a spiral jet mill, obtaining a dried micronized powder.

Said carrier is selected from the group comprising lactose, mannitol, glucose, trehalose, cellobiose, sorbitol, maltitol, colloidal silica, magnesium stearate, sucrose stearate, leucine, galactose, D-mannose, arabinose, sorbose, maltose, sucrose, xylitol, myo-inositol or erythritol, or a suitable excipient used as inhlation carrier, or a combination of two or more of them. In an embodiment, said carrier is lactose.

Said at least one mAb is in an aqueous solution, at a concentration comprised in the range 5 mg/ml - 100 mg/ml, or 8 mg/ml - 50 mg/ml, or 10 mg/ml - 30 mg/ml.

In an embodiment, said micronisation step is operated at the following parameters:

| |
|---|
| • Pressure: from 5 to 12 bar |
| • Process gas temperature: from -30°C to 25°C |

In an embodiment, said micronisation step is operated at the following parameters:

| |
|---|
| • Pressure: from 5 to 12 bar |
| • Process gas temperature: from -30°C to 25°C |

Feed rate: From 0.5 Kg/h to 24 Kg/h

In an embodiment, the granulation step includes adsorbing said solution comprising at least one mAb on said carrier, allowing the resulting wet powder mixture to dry and subjecting the dried powder mixture to a delumping process obtaining the dried granulated mixture.

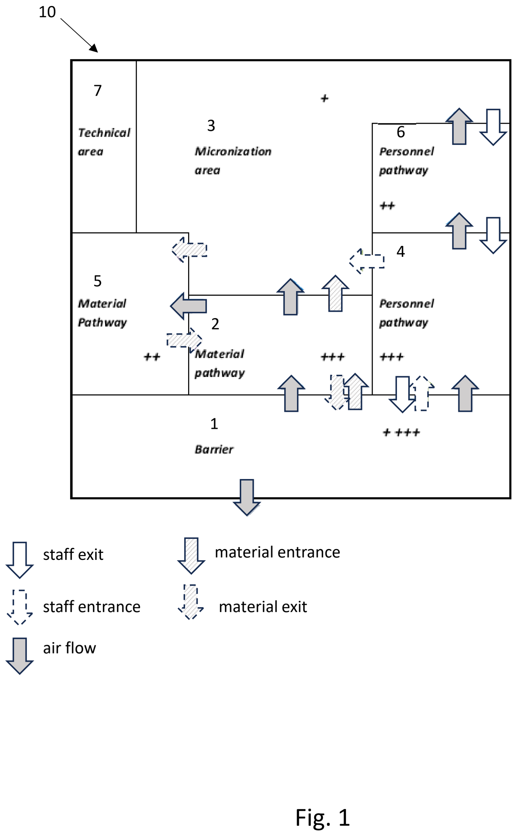

According to Figure 1, micronisation preferably occurs in a cleanroom (10), consisting of seven sections. The most external section is the barrier section (1) and prevents the product from leaking out of the working room and, at the same time, prevents air from the general area from entering the working room. Barrier section (1) is in overpressure (++++) with respect to the general production area and to the inner sections of the working cabin. Section (2) is the section for entrance/exit of the material: it is in overpressure with respect to the micronisation section (3) and it has a lower pressure than the barrier section (1). Section 4 is the section for entrance/exit of the personnel: it is in overpressure compared to the micronisation section (3) and it has a lower pressure (+) than the barrier section (1). Section (3) is the working area; it is in overpressure compared to the general production area and it has the lowest pressure compared to the other working cabin sections. Section (5) is the section for exit of the material, it is in overpressure (++) compared to the micronisation section (3) and it has a lower pressure than the barrier section (1) and the material entrance section (2). Section (6) is the section for exit of the personnel, it is in overpressure compared to the micronisation section (3) and it has a lower pressure than the barrier section (1) and the personnel entrance section (4). All the incoming air is filtered at least through HEPA 99.99% filtering systems and the working cabin has an independent air handling system.

The authors have surprisingly demonstrated that said method allows to obtain with a good efficiency, starting from an antibody in solution, a powder having a particle size suitable for DPI, while maintaining the biological activity of the antibody. The powder is stable over time.

The following examples are purely representative. They are not intended to limit the invention, whose scope of protection is defined by the claims that follow.

### Experimental data

The following equipment set up was engaged for the co-micronisation milling test:

| | |
|---|---|
| • Jet Mill | MC50, 4 inch spiral jet mill, AISI 316 |
| • Jet Mill | MCOne, 1 inch spiral jet mill, AISI 316 |
| • Feeder | K-Tron T-20, twin screw volumetric feeder, ASIS 316 |
| • Balance | Mettler scale PR503DR |
| • Filter bag | Antistatic polyester |

A co-micronised powder was obtained according to the method of the present invention for the antibodies listed in Table 1, starting from solution of said antibodies at the indicated concentrations:

**Table 1**

| **MAb** | **ID NO.** | **Concentration** |
|---|---|---|
| anti HER2 (Trastuzumab), liquid | Ab 1 | 20.5 mg/ml |
| anti CD20 (Rituximab), liquid | Ab 2 | 10 mg/ml |
| anti CD20 (Rituximab), liquid | Ab 3 | 20 mg/ml |
| anti HER2 (Trastuzumab), powder | Ab 4 | 420 mg trastuzumab + 322 mg sorbitol |

### Example 1: liquid Trastuzumab micronisation (Ab 1)

40 ml of the Ab1 solution were adsorbed drop by drop on 100 g of lactose un-milled powder. The resulting wet powder mixture was allowed to dry for one night. A delumping step was then applied, to homogenise the powder. The obtained powder was divided into 4 samples of about 25 g each. MC50 jet mill was used for the micronisation, at the following conditions:
- Experiment 1: Feed Rate = 5 g/30 sec.; Feeding Pressure = 5 bar; Grinding Pressure = 5 bar; Gas Temp. = -30°C
- Experiment 2: Feed Rate = 5 g/30 sec.; Feeding Pressure = 5 bar; Grinding Pressure = 5 bar; Gas Temp. = 18°C (room temp.)
- Experiment 3: Feed Rate = 5 g/30 sec.; Feeding Pressure = 10 bar; Grinding Pressure = 10 bar; Gas Temp. = -30°C
- Experiment 4: Feed Rate = 5 g/30 sec.; Feeding Pressure = 10 bar; Grinding Pressure = 10 bar; Gas Temp. = 18°C
3 samples were collected from each one of the micronized powders obtained from the 4 experiments. The samples were collected from left, center, right position for PSD analyses. The micronized powder left after sampling was packed inside a double LDPE bag, placed inside 3-layer aluminium bag with silica gel pack to protect from moisture.

The obtained results from the PSD analysis are reported in Table 2:

**Table 2**

| **Sample ID** | **Process conditions** | | | **PSD Results [µm]** | | |
|---|---|---|---|---|---|---|
| | **Feed Rate [Kg/h]** | **Pressure [bar]** | **Gas Temperature [°C]** | **X10** | **X50** | **X90** |
| Exp1 left | 0,6 | 5 | -30 | 0,85 | 3,67 | 8,59 |
| Exp1 center | 0,6 | 5 | -30 | 0,84 | 3,67 | 8,63 |
| Exp1 right | 0,6 | 5 | -30 | 0,86 | 3,67 | 8,51 |
| Exp2 left | 0,6 | 5 | 15-25 | 0,57 | 2,41 | 5,53 |
| Exp2 center | 0,6 | 5 | 15-25 | 0,57 | 2,47 | 5,75 |
| Exp2 right | 0,6 | 5 | 15-25 | 0,57 | 2,42 | 5,69 |
| Exp3 left | 0,6 | 10 | -30 | 0,64 | 2,47 | 5,38 |
| Exp3 center | 0,6 | 10 | -30 | 0,63 | 2,42 | 5,39 |
| Exp3 right | 0,6 | 10 | -30 | 0,64 | 2,49 | 5,38 |
| Exp4 left | 0,6 | 10 | 15-25 | 0,51 | 1,98 | 4,40 |
| Exp4 center | 0,6 | 10 | 15-25 | 0,52 | 1,99 | 4,41 |
| Exp4 right | 0,6 | 10 | 15-25 | 0,53 | 2,02 | 4,45 |

### Example 2: liquid Rituximab, 10 mg/ml, micronisation (Ab 2)

40 ml of the Ab 2 solution were adsorbed drop by drop on 200 g of lactose un-milled powder. The powder was allowed to dry for one night. A delumping step was then applied, to homogenise the powder. The obtained powder was divided into 2 samples of about 100 g each. MC50 jet mill was used for the micronisation, at the following conditions:
- Experiment 1: Feed Rate = 5 g/30 sec.; Feeding Pressure = 12 bar; Grinding Pressure = 12 bar; Gas Temp. = 20°C (room temp.)
- Experiment 2: Feed Rate = 5 g/30 sec.; Feeding Pressure = 12 bar; Grinding Pressure = 12 bar; Gas Temp. = -30°C

One sample was collected from each one of the micronized powders obtained from the 2 experiments. The micronized powder left after sampling was packed in a bottle, then placed inside 3-layer aluminium bag with silica gel pack to protect from moisture.

The obtained results from the PSD analysis are reported in Table 3:

**Table 3**

| **Sample ID** | **Process conditions** | | | **PSD Results [µm]** | | |
|---|---|---|---|---|---|---|
| | **Feed Rate [Kg/h]** | **Pressure [bar]** | **Gas Temperature [°C]** | **X10** | **X50** | **X90** |
| Exp1 | 0,6 | 12 | 15-25 | 0,48 | 1,79 | 4,12 |
| Exp2 | 0,6 | 12 | -30 | 0,49 | 1,89 | 4,39 |

### Example 3: liquid Rituximab, 20 mg/ml, micronisation (Ab 3)

20 ml of the Ab3 solution were adsorbed drop by drop on 100 g of lactose un-milled powder. The powder was allowed to dry for one night. A delumping step was then applied, to homogenise the powder. The obtained powder was divided into 2 samples of about 50 g each. MC50 jet mill was used for the micronization, at the following conditions:
- Experiment 1: Feed Rate = 5 g/30 sec.; Feeding Pressure = 5 bar; Grinding Pressure = 5 bar; Gas Temp. = -30°C
- Experiment 2: Feed Rate = 5 g/30 sec.; Feeding Pressure = 5 bar; Grinding Pressure = 5 bar; Gas Temp. = 15-25°C (room temp.)
- Experiment 3: Feed Rate = 5 g/30 sec.; Feeding Pressure = 10 bar; Grinding Pressure = 10 bar; Gas Temp. = -30°C
- Experiment 4: Feed Rate = 5 g/30 sec.; Feeding Pressure = 10 bar; Grinding Pressure = 10 bar; Gas Temp. = 15-25°C (room temp.)

One sample was collected from each one of the micronized powders obtained from the 2 experiments. The micronized powder left after sampling was packed in a bottle, then placed inside 3-layer aluminium bag with silica gel pack to protect from moisture.

The obtained results from the PSD analysis are reported in Table 4:

**Table 4**

| **Sample ID** | **Process conditions** | | | **PSD Results [µm]** | | |
|---|---|---|---|---|---|---|
| | **Feed Rate [Kg/h]** | **Pressure [bar]** | **Gas Temperature [°C]** | **X10** | **X50** | **X90** |
| Exp1 | 0,6 | 5 | -30 | 0.56 | 2.55 | 5.86 |
| Exp2 | 0,6 | 5 | 15-25 | 0.55 | 2.54 | 5.96 |
| Exp 3 | 0,6 | 10 | -30 | 0.51 | 2.00 | 4.48 |
| Exp 4 | 0,6 | 10 | 15-25 | 0.52 | 2.14 | 4.92 |

### Example 4: powder Trastuzumab micronisation (Ab 4, comparative)

Ab 4 was a lyophilized powder containing 420 mg Trastuzumab + 322 mg sorbitol. The micronisation process was performed with MCOne mill, a 1 inch spiral jet mill equipment made in AISI 316 stainless steel. Because of the small amount available we operated with manual feeding of the jet mill, without a feeder. The jet mill was fed with a lower as possible feed rate. The conditions were as follows:
- Experiment 1: Feed Rate = low.; Feeding Pressure = 12 bar; Grinding Pressure = 10 bar; gas Temp. = 20°C

One sample was collected from the micronized powder obtained. The micronized powder left after sampling was packed in a bottle, then placed inside 3-layer aluminium bag with silica gel pack to protect from moisture. The obtained results from the PSD analysis are reported in Table 5:

**Table 5**

| **Sample ID** | **Process conditions** | | | **PSD Results [µm]** | | |
|---|---|---|---|---|---|---|
| | **Feed Rate [Kg/h]** | **Pressure [bar]** | **Gas Temperature** [°C] | **X10** | **X50** | **X90** |
| Un-milled | NA | NA | NA | 8,79 | 36,41 | 97,76 |
| Microniza | 0,012 | 10 | 15-25 | 0,58 | 1,87 | 3,66 |

### Example 5: characterization of the micronized samples

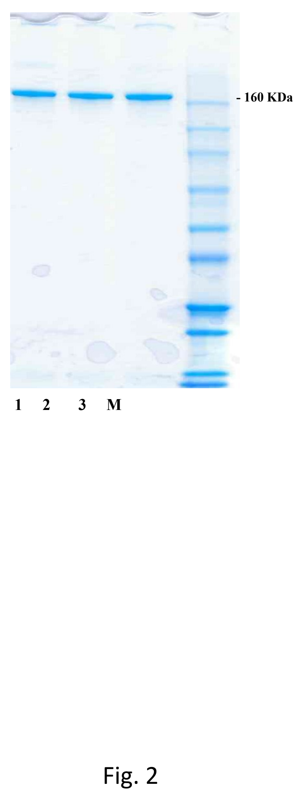

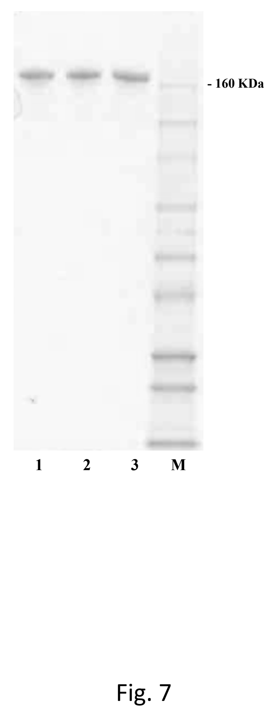

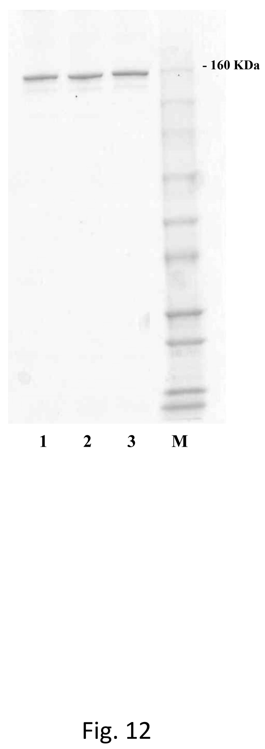

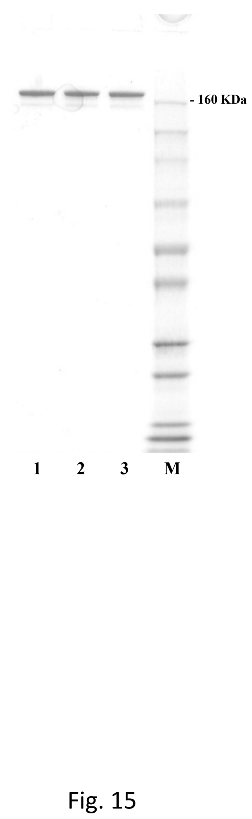

Antibodies after micronisation have the expected molecular weight of the reference product, as demonstrated in SDS-PAGE (Fig 2 Liquid Trastuzumab, Fig 7 Trastuzumab in powder, Fig 12 Rituximab 10 mg/ml and Fig 15 Rituximab 20 mg/ml).

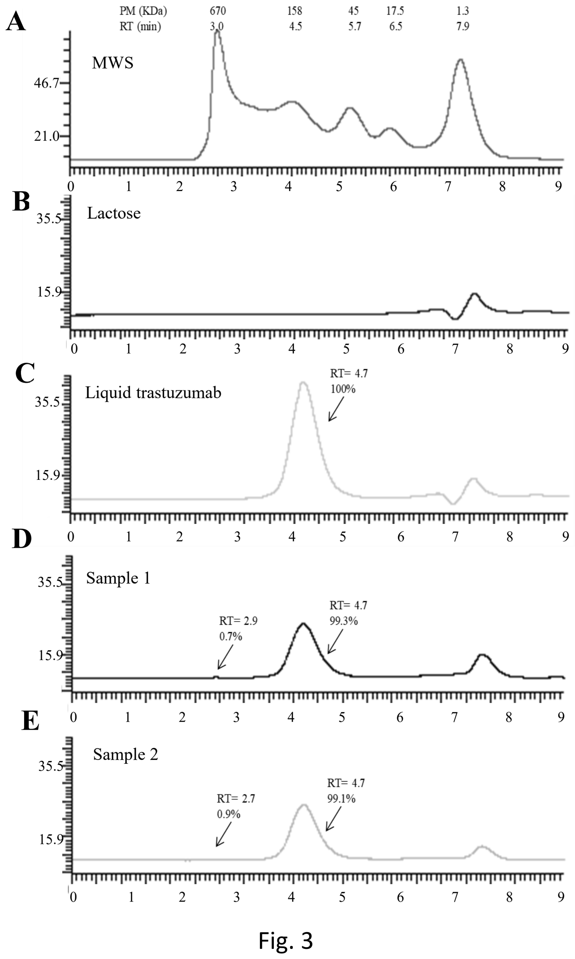

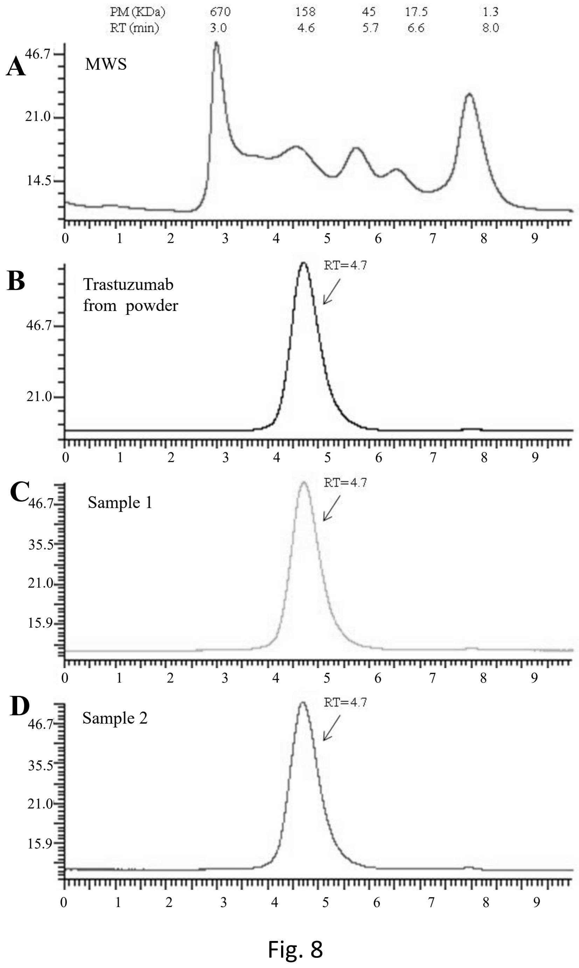

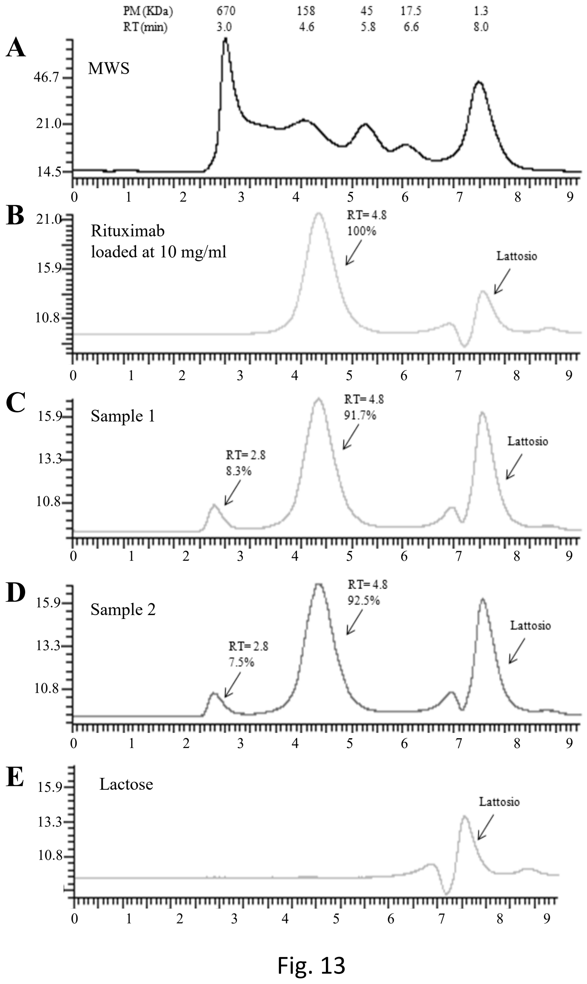

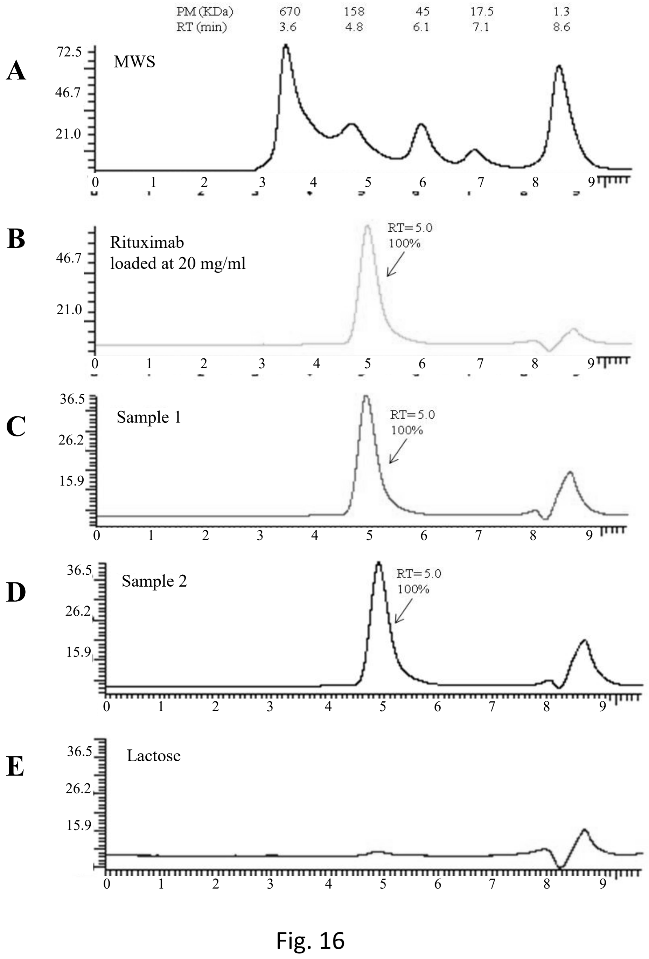

Size Exclusion Chromatography (SEC) is a well-established method to separate proteins and polymers by size and it is the method of choice for the determination of aggregates in the quality control of biopharmaceuticals. The SEC profiles obtained for the indicated samples (Fig 3 Liquid Trastuzumab, Fig 8 Trastuzumab in powder, Fig 13 Rituximab 10 mg/ml and Fig 16 Rituximab 20 mg/ml) indicate that the antibodies after micronisation do not aggregate; only an acceptable number of aggregates was present in the sample Rituximab 10 mg/ml. The problem was then solved using a more concentrate batch (Rituximab 20 mg/ml).

Measurements were performed on Series 200 HPLC system (Perkin Elmer) consisting of a solvent pump, an autosampler and a UV detector.

The data was analyzed with the TC Nav software. A Superdex 200 5/15 GL (15 x 5 mm) column (Cytiva) was used. A salt buffer was used consisting of 10 mM sodium hydrogen phosphate, 150 mM sodium chloride, adjusted to pH 7.4. An injection volume of 10 µl, a flow rate of 0.3 ml/min and a detection wavelength of 280 nm were applied.

### Example 6: functional analysis

Antigen binding of micronized antibodies was analyzed by FACS \ELISA and Surface Plasmon Resonance using Biacore T200.

### FACS (Fluorescence Activated Cell Sorter) testing

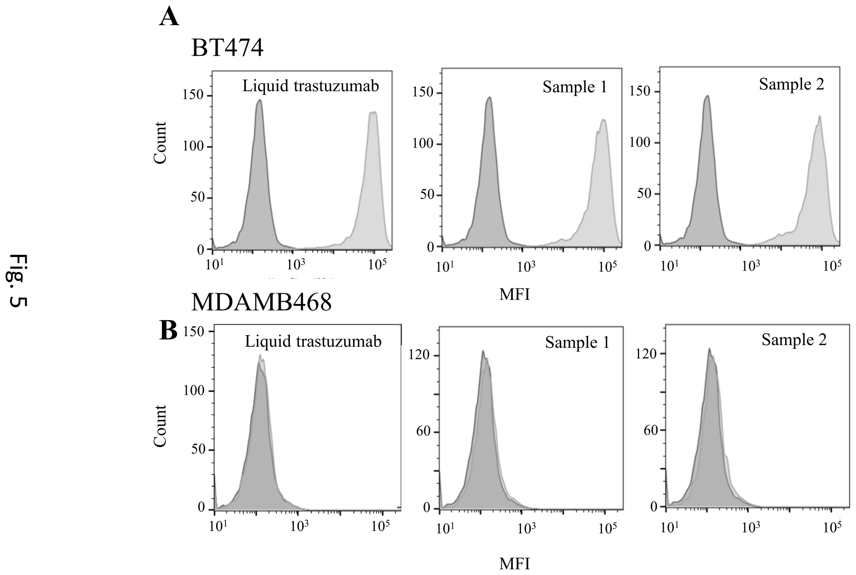

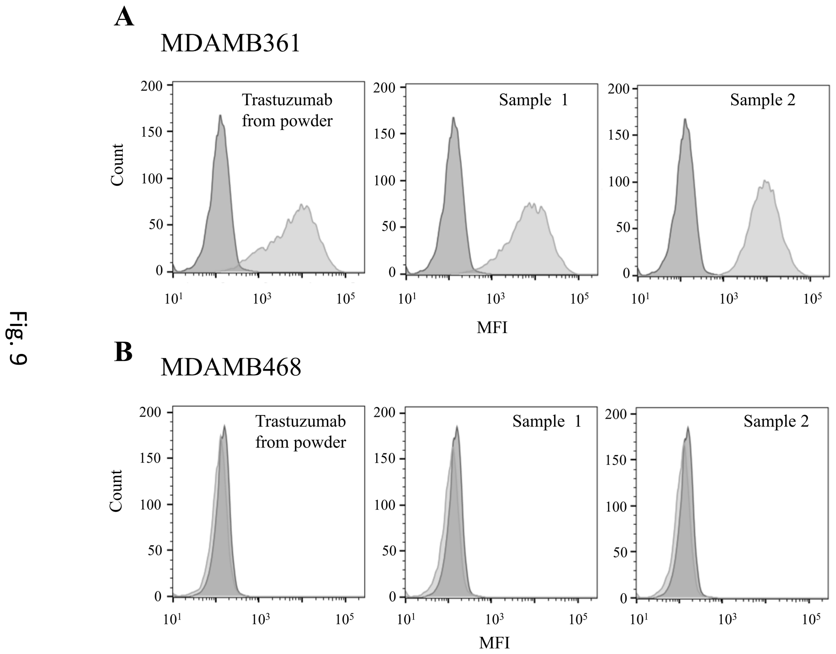

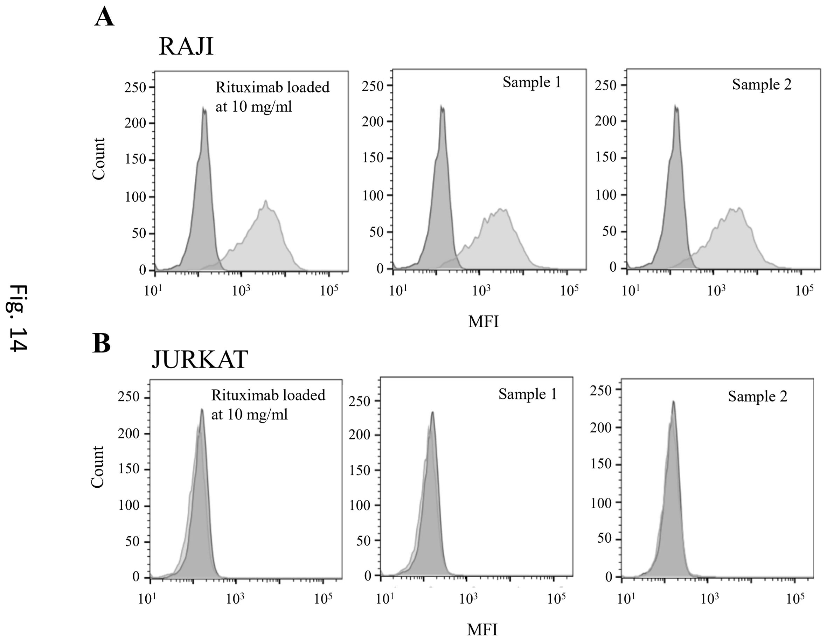

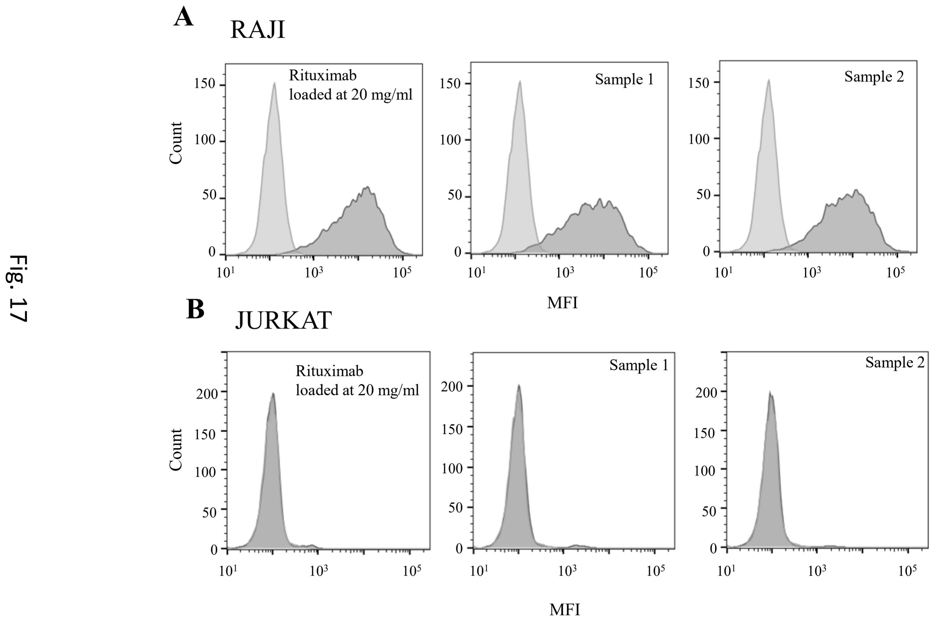

The micronized antibody or the reference antibody (10 µg/ml) were added to tumor cell (5×10⁵) in 100 µl PBS+ 0.03% BSA, and the mixture was incubated for 30 min on ice. The binding was detected by an antibody anti-human IgG-Alexa488 for 30 min on ice. For each sample, 5000 cells were analyzed with FACS Canto using DIVA software. The results are presented in Fig 5 Liquid Trastuzumab, Fig 9 Trastuzumab in powder, Fig 14 Rituximab 10 mg/ml and Fig 17 Rituximab 20 mg/ml. All the tested antibodies show specificity for the cells expressing (positive) or not expressing (negative) the antigen.

### ELISA (Enzyme-Linked Immunosorbent Assay) testing

5x10⁴ tumor cells were seeded on a 96-weel plate and fixed by glutaraldehyde (0.1% in PBS for 5 min) and glycine (0.1 M in PBS for 10 min).

The plate was saturated for 2 hours with BSA 1% in PBS.

Trastuzumab antibodies were tested in double and in dilution from 10 to 0.0025 µg/ml. The plate was incubated for one hour at room temperature. The binding was detected by an HPR-conjugated antibody anti-human IgG. Develop of the test with 100 µl of 3,3',5,5'-tetrametilbenzidina (TMB) and read at 450 nm after blocking with 50 µl of sulfuric acid 1 M.

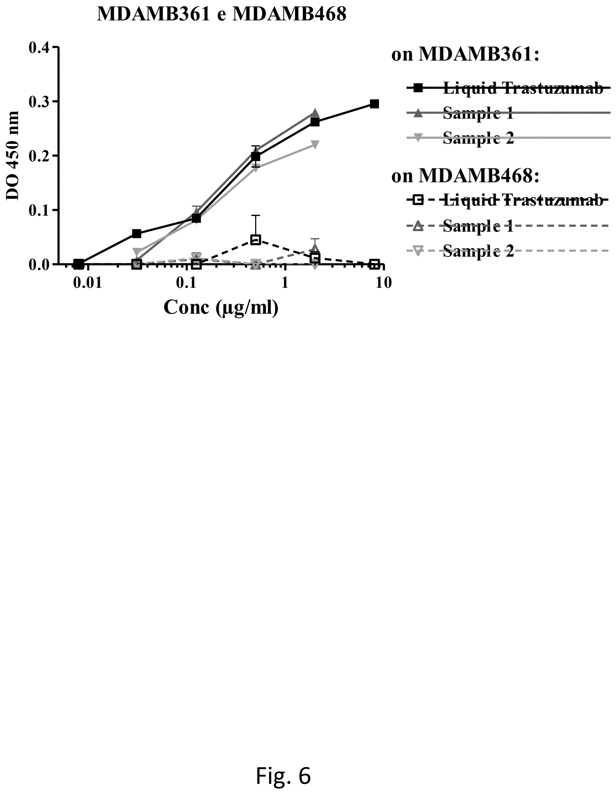

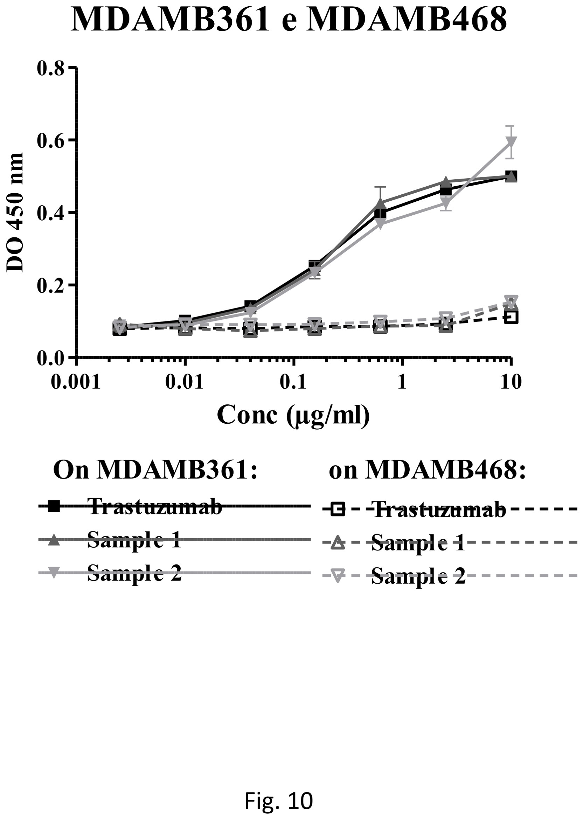

The results for Trastuzumab are reported in Figure 6 and Figure 10, showing the binding of the relative micronized antibodies on positive cells and on negative cells at different concentrations. The graphics show that the curves of the antibodies after micronisation present substantially the same binding behavior, within the experimental deviation limits, of the reference antibody; the same conclusion can be drawn for the set of curves relevant to negative cells.

### Surface Plasmon Resonance testing using Biacore T200

Biacore is an instrument based on Surface Plasmon Resonance for the characterization of biomolecular interactions, in this case antibodies and antigen (Jason-Moller L et al Curr Protoc Protein Sci. 2006 Sep; Chapter 19: Unit 19.13).

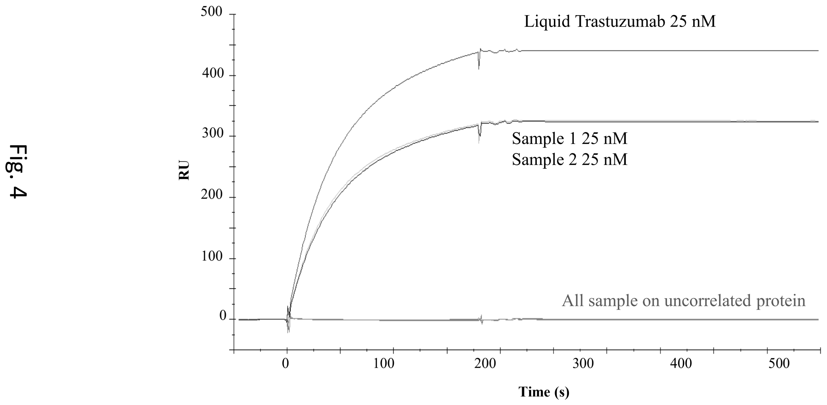

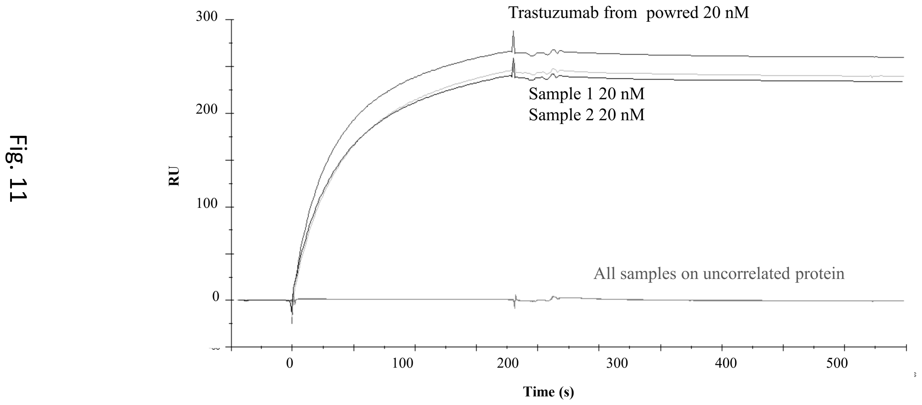

The micronized Trastuzumab and reference Trastuzumab were tested at concentration of 25 nM. The binding was carried out immobilizing the purified and soluble antigen; (hHER2-ECD) on sensorchip CM5 (Figure 4 and Figure 11).

Table 6 is a summary of the results obtained when micronizing the selected antibodies at the indicated concentrations.

**Table 6**

| **Antibo dy** | **Micronizat ion conditions** | **% of aggregat es** | **Fragme nts** | **Functionality** (% = Ab sample on Ab in lactose) | | | **% non specif ic bindi ng** |
|---|---|---|---|---|---|---|---|
| | | | | **ELIS A** | **FAC S** | **BIACO RE** | |
| **Ab1** | 5 bar/ -30°C | 2.7 | No | 100 | 81 | 88 | 0 |
| | 5 bar/ 18°C | 3.1 | No | 91 | 98 | 82 | 0 |
| | 10 bar/ - 30°C | 2.8 | No | 83 | 104 | 78 | 0 |
| | 10 bar/ 18°C | 3.5 | No | 95 | 105 | 73 | 0 |
| **Ab4** | 10 bar/ 20°C | 0 | No | 105 | 109 | 91 | 0 |
| **Ab2** | 12 bar/ 20°C | 7.9 | No | ND | 96 | ND | 0 |
| | 12 bar/ - 30°C | 7.9 | No | ND | 103 | ND | 0 |
| **Ab3** | 5 bar/ -30°C | 3.8 | No | ND | 141 | ND | 0 |
| | 5 bar/ 20°C | 3.3 | No | ND | 132 | ND | 0 |
| | 10 bar/ - 30°C | 3.8 | No | ND | 127 | ND | 0 |
| | 10 bar/ 20°C | 5.6 | No | ND | 130 | ND | 0 |

The procedure according to the present invention simplifies the process. The two steps, granulation and micronisation, are more sustainable than the freeze-drying process followed by micronisation currently in use.

The process works with all the tested antibodies. The relevant parameter is the initial concentration of the antibody, to improve the quality of the final product in term of aggregates.

Moreover, the obtained powder is more flowable, packs less, and it is less bulky than the one obtained after the freeze-drying process.
This allows a more robust process, wherein a powder having said characteristics allows an easier dosing management in jet mill.

## Claims

1. A method of making a micronized powder for inhalation comprising the step of:
- Granulating an un-milled carrier with a solution comprising at least one monoclonal Antibody (mAb) to obtain a dried granulated mixture;
- Micronizing the dried granulated mixture with a spiral jet mill,
- Obtaining a micronized powder.

2. The method according to claim 1, wherein said carrier is selected in the group comprising lactose, mannitol, glucose, trehalose, cellobiose, sorbitol, maltitol, colloidal silica, magnesium stearate, sucrose stearate, leucine, galactose, D-mannose, arabinose, sorbose, maltose, sucrose, xylitol, myo-inositol or erythritol, or a suitable inhalation carrier, or a combination of two or more of them.

3. The method according to claim 2, wherein said carrier is lactose.

4. The method according to any one of the claims 1-3, wherein said at least one mAb is in an aqueous solution, at a concentration comprised in the range 5 mg/ml - 100 mg/ml, or 8 mg/ml - 50 mg/ml.

5. The method according to any one of the claims 1-4, wherein said at least one mAb is in an aqueous solution, at a concentration comprised in the range 10 mg/ml - 30 mg/ml.

6. The method according to any one of the claims 1-5, wherein the granulating step includes adsorbing said solution comprising at least one mAb on said carrier, allowing the resulting wet powder mixture to dry and subjecting the dried powder mixture to a delumping process obtaining the dried granulated mixture.

7. The method according to any one of the claims 1-6, wherein said micronisation is performed using the following parameters:
- Pressure: from 5 to 12 bar
- Process gas temperature: from -30°C to 25°C.

8. The method according to any one of the claims 1-6, wherein said micronisation is performed using the following parameters:
- Pressure: from 5 to 12 bar
- Process gas temperature: from -30°C to 25°C.
- Feed rate: From 0.5 Kg/h to 24 Kg/h
